# EUROPEAN PATENT SPECIFICATION

(11) **EP 0 711 171 B1**
(45) Date of publication and mention of the grant of the patent: **04.09.2002**
(21) Application number: 94917019.5
(22) Date of filing: 13.06.1994
(51) Int. Cl.: A61K 35/20, A61P 17/02

(54) **A PHARMACEUTICAL OR COSMETIC COMPOSITION COMPRISING A COLOSTRUM FRACTION AND ITS MEDICAL USE**
EINE PHARMAZEUTISCHE ODER KOSMETISCHE ZUBEREITUNG, DIE EINE KOLOSTRUMFRAKTION ENTHÄLT UND DEREN MEDIZINISCHE VERWENDUNG
COMPOSITION PHARMACEUTIQUE OU COSMETIQUE CONTENANT UNE FRACTION DE COLOSTRUM ET SON UTILISATION MEDICALE

(30) Priority: 23.06.1993 US 80218
(43) Date of publication of application: 15.05.1996
(73) Proprietor: Novatreat Oy, 20750 Turku (FI)
(72) Inventor: LAATO, Matti Kalevi, FIN-21270 Nousiainen (FI); JALKANEN, Markku Tapani, FIN-20760 Piispanristi (FI); JALONEN, Harry Gösta, FIN-20610 Turku (FI); AALTO, Jouni Uolevi, FIN-21260 Raisio (FI); KANTTINEN, Ari Perttu Tapani, FIN-20500 Turku (FI); PAKKANEN, Raimo Antero, FIN-21110 Naantali (FI)
(74) Representative: Öhman, Ann-Marie
(86) International application number: FI9400252
(87) International publication number: WO95000155

(56) References cited:
- EP-A- 0 334 776
- FI-A- 893 404
- FR-A- 2 460 135
- FR-A- 2 627 386
- US-A- 4 342 747
- DERWENT ABSTRACT, No. 92-248203/30, week 9230; & SU,A,1685253 (KOZLOV V K), 15 October 1991.

## Description

### FIELD OF INVENTION

This invention relates to a pharmaceutical or cosmetic composition comprising a colostrum fraction and use thereof.

### BACKGROUND OF THE INVENTION

The term "improving the healing of wounds" in the present text refers to the taking of steps by means of which wounds showing no tendency to heal are induced to start the healing process, and wounds that have started to heal are induced to heal more quickly; or to the taking of steps which are conductive to a cosmetic improvement of the functional result during the healing or after the completion thereof. Improvement of wound healing is particularly important when natural healing is slow or is rendered difficult by a number of negative factors like e.g. infection of the wound, impeded blood flow, medical treatments with cell poisons or with steroids of various kinds, or in cases where patients suffer from chronical disorders with concomitant impairment of normal wound healing e.g. when they are bedridden for prolonged periods of time or suffering from burns, hypertrophic scars, keloids, old age, cancer, diseases giving rise to serious nutritional deficiencies of such types as e.g. chronical inflammatory conditions of the intestine, conditions caused by extensive bodily injuries (so-called polytrauma patients), and comparable type of conditions.

Factors stimulating the healing of wounds have been described earlier. As examples can be mentioned the epidermal growth factor (EGF), platelet derived growth factor (PDGF) (Grotendorst, J Clin Invest 76:2323, 1985). These factors are known to produce an increased cell division in organ cultures. EGF was thought at the outset to be a promising healing stimulant when tested in experimental studies of its effects in vivo.

The main cell types of skin consists of epidermal keratinocytes and dermal fibroblasts. For wound healing it would be beneficial if the growth and/or migration of epidermal keratinocytes could be promoted. Also for the general skin care keratinocyte growth stimulation can help to maintain the healthy look of skin.

Many known growth factors and other agents known to stimulate the epidermal growth suffer from the disadvantage that they stimulate the growth of the connective (granulation) tissue too strongly compared to their stimulating influence on epidermal (keratin) layer. Excessive collagen synthesis could lead to hypertrophic scars or keloids, such as e.g. in burns. An ideal result would be obtained if the keratin layer grows rapidly while the granulation tissue is growing very slowly or not growing at all. An optimal stimulating agent should therefore exhibit a high growth promoting effect on the keratin layer and a growth inhibiting effect on the granulation tissue.

The European patent publication EP 334776 concerns cosmetic or pharmaceutical compositions comprising microfiltrated or ultrafiltrated colostrum serum as ingredient. The pore size of the filter is preferably about 0.14 micrometer. In the publication the compositions are said to have a hydrating, stimulating, softening, nourishing, regenerating and cicatrising action. No wound healing effect is disclosed.

The patent publications FR 2460135 and US 4342747 relate to the use of colostrum from which cream has been removed for the use as an ophthalmic agent. They do not relate to a colostrum fraction obtained as filtrate by filtering colostrum through a 100 000 Da membrane nor do they relate to the use of such a fraction for wound healing.

The Russian patent publication SU 1685253 relates to the use of the fat fraction of colostrum.

The patent publication FR 2627386 concerns compositions of colostrum lactoserum and vitamin A for ocular use. No ultrafiltration with a membrane cut off of 100 000 Da is disclosed. No effect on wound healing is either disclosed.

The patent publication WO 93/08264 discloses a colostrum fraction having a low endotoxin, protein and immunoglobulin concentration prepared by ultrafiltration of pretreated bovine colostrum, using a membrane having a cut off of 100,000 Da.

### SUMMARY OF THE INVENTION

This invention relates to a pharmaceutical or cosmetic composition comprising a colostrum fraction, said colostrum fraction having been prepared by subjecting colostrum, from which part of the fat and cellular debris have been removed by conventional methods, to ultrafiltration by using a membrane having a cut off of 100,000 Da and recovering the filtrate, and a compatible non-toxic pharmaceutical or cosmetic carrier therefor. This invention also relates to the use of said fraction for the manufacture of a pharmaceutical or cosmetic composition.

### BRIEF DESCRIPTION OF THE DRAWINGS

Figures 1A and 1B demonstrate transdermal preparations.

Figure 2 demonstrates the stimulation of keratinocyte DNA synthesis by different concentrations of colostrum fraction.

Figure 3 discloses proliferation of HaCat cells in vitro in 10 % FCS (triangles), 1 % adult bovine serum (circles) and 1 % adult bovine serum + 5 % CF (squares).

### DESCRIPTION OF THE PREFERRED EMBODIMENTS

The inventors have found that a colostrum fraction containing the growth factors of colostrum stimulate the growth of the keratin layer very strongly while it simultaneously inhibits the growth of the granulation tissue.

Bovine colostrum fractions rich in growth factors are known as excellent additives to cell culture media to promote the growth of cells, particularly hybridoma cells. Reference is made to R Pakkanen et al, Appl Microbiol Biotechnol (1992) 37: 451-456 and International Patent Application No PCT/FI92/00268. These publications describe the preparation of the colostrum fraction which has very low concentrations of endotoxin, protein and immunoglobulin but which contains growth factors, trace elements, vitamins and other small-molecular compounds essential for cell growth present in colostrum.

The invention thus concern a method for improving the healing of wounds in mammals including humans which comprises locally administering to said mammal a safe and effective amount of a colostrum fraction, said colostrum fraction having been prepared by subjecting colostrum, from which part of the fat and cellular debris have been removed by conventional methods such as centrifugation, to ultrafiltration by using a membrane having a cut off of 100,000 Da and recovering the filtrate.

The invention further concerns a pharmaceutical or cosmetic composition, particularly a composition for improving the healing of wounds in mammals including humans comprising a colostrum fraction, said colostrum fraction having been prepared by subjecting colostrum, from which part of the fat and cellular debris have been removed by conventional methods, to ultrafiltration by using a membrane having a cut off of 100,000 Da and recovering the filtrate, and a compatible non-toxic carrier therefor.

According to a preferred embodiment, casein has been removed from the colostrum by precipitation previous to its ultrafiltration. The removal of casein results in lower endotoxin and immunoglobulin contents in the ultrafiltrated colostrum fraction.

The colostrum fraction is for practical reasons preferably obtained from bovine colostrum. Colostrum from other sources is, however, also believed to be useful for the preparation of the colostrum fraction to be used in this invention.

The topical or transdermal administration of the colostrum fraction can be accomplished mainly in three different ways: (i) by mixing (or adsorbing) an effective amount of the colostrum fraction with suitable non-toxic carriers and optionally penetration enhancers to form ointments, emulsions, solutions, suspensions, lotions, creams, gels, powders, aerosols, bandage patches or the like, where preferably a fixed amount of said formulation is to be applied onto the wound, or (ii) by incorporating the colostrum fraction into a transdermal delivery system according to known technology, or (iii) by incorporating the bioactive substances from the colostrum fraction into liposomes which can either be used as such or can be incorporated in the formulations of the previous alternatives (i) or (ii).

In the compositions of this invention, the role of the transdermal administration is mainly to transport the colostrum fraction through the epidermal layer and not necessarily to achieve a systemic effect.

Examples of suitable excipients include those well known in the art of pharmacy and cosmetology for the preparation of topical formulations such as aqueous buffered saline solutions, non-volatile fatty alcohols, acids, esters, e.g. cetostearyl alcohol and cetyl alcohol; volatile alcoholic compounds, e.g. ethanol and isopropanol; glycols and glycol ethers.

The healing of wounds in the stomach or the intestine, i.e. peptic ulcers can also be improved by use of the method according to the invention. For the treatment of peptic ulcers the colostrum fraction can be used as such combined with flavouring agents and preservatives. Alternatively, the colostrum fraction can be absorbed into particles or into a matrix, which optionally could be coated with a material having slow release or enteric properties. The matrix or particles can further be pressed into tablets or put into capsules.

The following non-limiting examples demonstrate the compositions according to the invention.

### Reference example

Whey was prepared from defatted colostrum by removal of casein using acid precipitation, (see, for example, R. Pakkanen et al., Appl. Microbiol Biotechnol (1992) 37; p. 452.). An ultrafiltrate was obtained from cleared whey by filtration throuhg membranes with a nomonal molecular weight cut-off of 100,000 Da. The colostrum ultrafiltrate (colostrum fraction) so obtained contained 1.1g g/l protein, 0.24 g/l immunoglobulin G (IgG) and less than 0.24 EU (endotoxin unit)/ml endotoxins.

### Example 1

### Examples of semisolid preparations

a)

| | |
|---|---|
| White petrolatum | 20 g |
| Stearyl alcohol | 25 g |
| Propylene glycol | 10 g |
| Sodium lauryl sulfate | 1 g |
| Methyl paraben | 25 mg |
| Propyl paraben | 15 mg |
| Distilled water | 34 ml |
| Colostrum fraction | 10 ml |

b)

| | |
|---|---|
| Polyethylene glycol 4000 | 45 g |
| Polyethylene glycol 400 | 45 g |
| Colostrum fraction | 10 ml |

c)

| | |
|---|---|
| Methocel 90 H.C. 4000 | 1 g |
| Carbopol 934 | 24 mg |
| Propylene glycol | 18 g |
| Methyl paraben | 15 mg |
| Distilled water | 71 ml |
| Colostrum fraction | 10 ml |
| NaOH added to adjust the pH to 7. | |

### Example 2

### Transdermal formulations

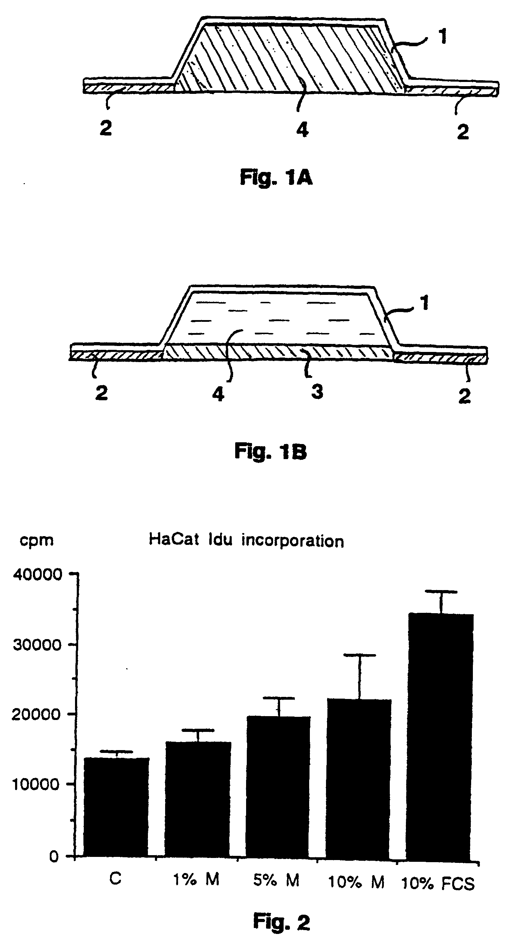

The colostrum fraction can be incorporated into a transdermal drug delivery device. Such devices are illustrated in Fig. 1A and 1B, where reference number 1 represents an backing layer, 2 is an adhesive layer, which keeps the patch attached to the skin and 3 is a porous polymer. In Fig. 1A the colostrum fraction has been absorbed into the porous polymer 3, either alone or in combination with a vehicle and/or a penetration enhancer. In Fig. 1B the reservoir 4 contains the colostrum fraction solution.

### Example 3

### Liposome formulations

| | |
|---|---|
| Colostrum fraction | 1 ml |
| Phosphatidyl choline | 70 mg |
| Phosphatidyl serine | 20 mg |
| Cholesterol | 10 mg |
| Buffered distilled water | 10 ml |

The best way to prepare liposomes of the above mixture is either the use of the French press or the membrane extrusion method. Alternatively, it is possible to prepare unloaded liposomes according to known methods and later add the bioactive components of the colostrum fraction by using pH gradients according to the active loading principles (R R C New, Liposomes - a practical approach, IRL Press at Oxford University Press).

The required dose of the colostrum fraction will vary especially with the particular kind of wound to be treated, the severity of its condition and the dosage form. We believe that a suitable dose could range from some millilitres to one litre or more of colostrum fraction per day and adult person. The preferred dose for topical or transdermal preparations would be about 0.5 - 1 ml/day and cm² of wound area. For the treatment of peptic ulcers the preferable oral dosage is believed to be about 50 to 1000 ml per day and adult person.

The therapeutic effect of the colostrum fraction is demonstrated by the following non-limiting examples. The colostrum fraction used in the experiments had an endotoxin content of 1.0 EU/ml, a total protein content of 2.0 mg/ml and an immunoglobulin content of 0.25 mg/ml. The fraction can was obtained by subjecting the defatted colostrum to casein precipitation before the ultrafiltration.

### Example 4

### Treatment of keratinocyte cultures with colostrum fraction (CF) solutions

The effect of the colostrum fraction (CF) was therefore studied on keratinocyte growth stimulation. This was done with classical cell culture method, where cultured keratinocytes were exposed to various concentration of CF. A human keratinocyte cell line (HaCat: Fusenig and Worst Exp. Cell Res. 93:443-457, 1992) was cultured routinely in modified Eagle's MEM supplemented with 10 % FCS (fetal calf serum) and antibiotics at 37°C, 5 % CO₂ and 95 % air in a humidified incubator as described (Elenius et al. J. Biol. Chem., 265: 17837-17843, 1990). In the first experiment, HaCat cells were divided into petri dishes and grown until semi-confluent. After that cells were maintained 24 h in serum free medium following the addition of different concentrations of CF and FCS. Cells were incubated with these solutions for next 24 h after which ¹²⁵I-deoxiuridine was added for an additional 2 hours. Finally cells were washed with cold PBS, fixed and extracted into 0.2 M NaOH.

Radioactivity remaining in the cell extracts was measured with a gamma counter.

Figure 2 demonstrates the stimulation of keratinocyte DNA synthesis by CF. Human keratinocytes were plated on culture dishes and grown to semiconfluency in the presence of 10 % FCS. After 24-h starvation in plain culture medium, media containing different concentrations of CF or 10 % FCS were added for following 24 hours. At the end of the incubation DNA synthesis rates were measured by adding ¹²⁵I-deoxyuridine into cultures for additional two hours and measuring the rates of IdU incorporations. Mean ± S.D. of four samples are presented. C = plain medium; 1 % M = 1 % CF; 5 % M = 5 % CF; 10 % M = 10 % CF; 10 % FCS = 10 % fetal calf serum.

As shown in Figure 2, this analysis revealed a stimulation of DNA synthesis in the presence of increasing concentrations of CF. This stimulation was almost as high as stimulation obtained with fetal calf serum, the best growth supported for cultured cells. These results clearly indicate that CF contains factors which can stimulate DNA synthesis of keratinocytes.

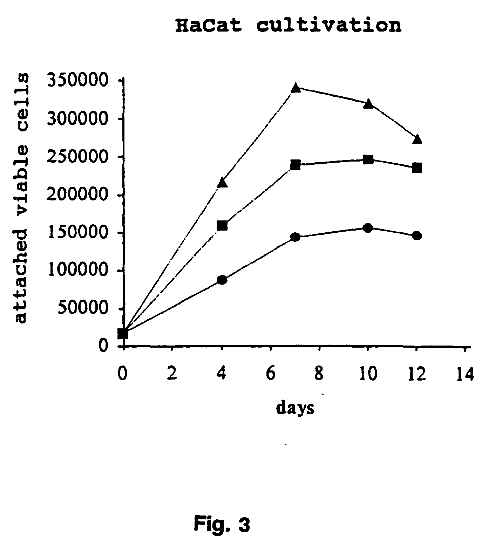

In the second experiment HaCat cells were suspended in modified Eagle's MEM supplemented with 1 % adult bovine serum and antibiotics and divided into 24-well cell culture plates at a concentration of 30 000 viable cells/ml (1.5 ml/well). The cells were incubated at 37°C, 5 % CO₂ and 95 % air for 24 hours. Then, the media were removed from the wells and 1.5 ml samples of test media were added (10 % FCS, 1 % adult bovine serum, 5 % CF + 1 % adult bovine serum in modified Eagle's MEM supplemented with antibiotics). At the time points indicated (Fig. 3) the attached cells were detached with trypsin and counted. The old media samples of the other wells were also replaced with fresh samples at the same days. Cell counts were performed in a haemocytometer using trypan blue exclusion to determine viability and each cell was counted only once. The experiment was performed in duplicate.

As shown in Fig. 3 the results indicate that CF contains growth factors which stimulate proliferation of HaCat cells in vitro. Moreover, the results suggest that these factors are either not present in normal adult bovine serum or their concentration is significantly higher in CF.

### Example 5

### Effect of the colostrum fraction on the granulation tissue.

The effect of the colostrum fraction (CF) as described in the foregoing example on the wound healing improvement was studied in a standardized experimental wound model in rats as described by Niinikoski, Heughan and Hunt (Surg Gynecol Obstet 133: 1003-1007, 1971). This model could briefly be described as follows:

### Treatment of the wound with solutions of a colostrum fraction (CF):

Viscose cellulose sponge (Säteri Oy, Valkeakoski, Finland) was used as an inductive matrix for repair tissue. The material was cut into cylindrical pieces, 40 mm long and 10 mm in diameter, and a tunnel of 3 mm in diameter was made through the center of the sponge. Silicone rubber discs, 10 mm in diameter and 2 mm thick, were stiched onto both ends of the sponge to create a stable dead space. The cylinders were decontaminated by boiling for 30 min in physiological saline and the implantations were performed with strictly aseptic techniques. Male Spraque Dawley rats weighing 230 - 250 g were anesthetized with ether and an incision, 4 cm long, was made in the dorsal midline at the caudal portion of the back. Each rat received one sponge cylinder that was implanted longitudinally under the skin, cephalad from the incision. During the experiments the animals received a normal rat diet and water ad libitum and were housed individually in cages in the animal quarters. The design of the work was approved by the local ethical committee.

### In vivo experiments:

Altogether 32 rats were studied in four groups of 8 animals. In the three test groups the implants were injected immediately after implantation with 1 ml of 1, 5 or 10% of colostrum fraction (CF) in phosphate buffer saline (PBS) into the central tunnel. The control group was injected similarly with the carrier solution only. Injections of all the groups were repeated daily under strictly aseptic conditions. No infections were observed. After collection of the wound fluid samples the rats were anesthetized with ether and sacrified. The implants were dissected free from the surrounding tissue, and the silicone rubber discs were removed. Nucleic acids were extracted from the implants according to the method of Schmidt and Thannhauser. DNA was determined by the diphenylamine reaction and RNA was assayed as RNA-ribose by the method of Ceriotti. Aliquots were taken for the determination of nitrogen, hydroxyproline, hexosamines and uronic acids (Laato M, Acta Chir Scand Suppl 546: 1-44, 1988).

The results are given in Table 1. The units are mg/sponge and the abbreviations have the following meaning: CF = colostrum fraction; HYPRO = hydroxypropyline; HEXOS = hexosamines and URONIC = uronic acid.

**Table 1**

| | DNA | RNA-Ribose | NITROGEN | HYPRO | HEXOS | URONIC |
|---|---|---|---|---|---|---|
| Groups | Average | Average | Average | Average | Average | Average |
| | | | | | | |
| control | 33,825 | 5,5625 | 82,6 | 10,005 | 6,875 | 6,825 |
| 1% CF | 36,125 | 6,0375 | 82,5625 | 9,725 | 6,575 | 7,2625 |
| 5% CF | 28,275 | 6,5375 | 89,9125 | 7,13 | 5,2875 | 6,2375 |
| 10% CF | 25,8875 | 6,5875 | 83,425 | 8,25 | 6,375 | 6,8 |
| | | | | | | |

| | P-value | P-value | P-value | P-value | P-value | P-value |
|---|---|---|---|---|---|---|
| | 0,01285 | 0,00515 | 0,18347 | 0,24312 | 0,03023 | 0,07885 |

## Claims

1. A pharmaceutical or cosmetic composition comprising a colostrum fraction, said colostrum fraction having been prepared by subjecting colostrum, from which part of the fat and cellular debris have been removed by conventional methods, to ultrafiltration by using a membrane having a cut off of 100,000 Da and recovering the filtrate, and a compatible non-toxic pharmaceutical or cosmetic carrier therefor.

2. The composition according to claim 1 being useful for the improvement of the healing of wounds in mammals including humans.

3. The composition according to claim 2 wherein casein has been removed from the colostrum by precipitation previous to ultrafiltration.

4. The composition according to claim 3 wherein the colostrum fraction is a bovine colostrum fraction.

5. The composition according to claim 2 which is in the form of ointment, emulsion, suspension, lotion, solution, gel, cream, powder or aerosol.

6. The composition according to claim 2 which is a transdermal delivery system.

7. The composition according to claim 2 which is in the form of a tablet or capsule for oral administration.

8. The use of a colostrums fraction, said colostrum fraction having been prepared by subjecting colostrum, from which part of the fat and cellular debris have been removed by conventional methods, to ultrafiltration by using a membrane having a cut off of 100,000 Da and recovering the filtrate, for the manufacture of a pharmaceutical or cosmetic composition for healing wounds in mammals including humans.

## Patentansprüche

1. Pharmazeutische oder kosmetische Zusammensetzung, umfassend eine Kolostrum-Fraktion, wobei die Kolostrum-Fraktion hergestellt wurde, indem Kolostrum, aus dem ein Teil des Fetts und Zelltrümmer durch herkömmliche Verfahren entfernt worden waren, einer Ultrafiltration durch Verwendung einer Membran mit einem Cut-off-Wert von 100.000 Da unterworfen wurde und das Filtrat gewonnen wurde, und einen kompatiblen nichttoxischen pharmazeutischen oder kosmetischen Träger dafür.

2. Zusammensetzung nach Anspruch 1, die zur Verbesserung der Heilung von Wunden bei Säugern einschließlich Menschen verwendbar ist.

3. Zusammensetzung nach Anspruch 2, wobei Casein vor einer Ultrafiltration durch Präzipitation aus dem Kolostrum entfernt wurde.

4. Zusammensetzung nach Anspruch 3, wobei die Kolostrum-Fraktion eine Rinder-Kolostrum-Fraktion ist.

5. Zusammensetzung nach Anspruch 2, die in Form einer Salbe, Emulsion, Suspension, Lotion, Lösung, eines Gels, einer Creme, eines Pulvers oder eines Aerosols vorliegt.

6. Zusammensetzung nach Anspruch 2, die ein transdermales Abgabesystem ist.

7. Zusammensetzung nach Anspruch 2, die in Form einer Tablette oder einer Kapsel zur oralen Verabreichung vorliegt.

8. Verwendung einer Kolostrum-Fraktion, wobei die Kolostrum-Fraktion hergestellt wurde, indem Kolostrum, aus dem ein Teil des Fetts und Zelltrümmer durch herkömmliche Verfahren entfernt worden waren, einer Ultrafiltration durch Verwendung einer Membran mit einem Cut-off-Wert von 100.000 Da unterworfen wurde und das Filtrat gewonnen wurde, zur Herstellung einer pharmazeutischen oder kosmetischen Zusammensetzung zur Heilung von Wunden bei Säugern einschließlich Menschen.

## Revendications

1. Composition pharmaceutique ou cosmétique comprenant une fraction de colostrum, ladite fraction de colostrum ayant été obtenue en soumettant le colostrum, dont on a éliminé une partie de la graisse et des débris cellulaires par des méthodes classiques, à une ultrafiltration au moyen d'une membrane ayant un seuil de coupure de 100 000 dalton et en récupérant le filtrat, et un véhicule non toxique acceptable en pharmacie ou en cosmétique pour ceci.

2. Composition selon la revendication 1, utilisable pour améliorer la guérison des plaies chez les mammifères, y compris l'homme.

3. Composition selon la revendication 2, dans laquelle la caséine a été éliminée du colostrum par précipitation préalablement à l'ultrafiltration.

4. Composition selon la revendication 3, dans laquelle la fraction de colostrum est une fraction de colostrum bovin.

5. Composition selon la revendication 2, qui se présente sous la forme d'un onguent, d'une émulsion, d'une suspension, d'une lotion, d'une solution, d'un gel, d'une crème, d'une poudre ou d'un aérosol.

6. Composition selon la revendication 2, qui est un système de libération transdermique.

7. Composition selon la revendication 2, qui se présente sous la forme d'un comprimé ou d'une capsule destiné à l'administration par voie orale.

8. Utilisation d'une fraction de colostrum, ladite fraction de colostrum ayant été obtenue en soumettant le colostrum, dont on a éliminé une partie de la graisse et des débris cellulaires par des méthodes classiques, à une ultrafiltration au moyen d'une membrane ayant un seuil de coupure de 100 000 dalton et en récupérant le filtrat, pour la fabrication d'une composition pharmaceutique ou cosmétique destinée à la guérison des plaies chez les mammifères, y compris l'homme.
